Europäisches Patentamt

European Patent Office

Office européen des brevets

(11) Publication number: **0 071 980**
**B1**

## (12) EUROPEAN PATENT SPECIFICATION

(45) Date of publication of patent specification: **25.11.87**

(21) Application number: **82107058.8**

(22) Date of filing: **04.08.82**

(51) Int. Cl.⁴: **A 61 B 5/00,** A 61 B 5/02, A 61 B 5/08

(54) **Sensing device for cardio-pulmonary functions.**

(30) Priority: **10.08.81 JP 124180/81**

(43) Date of publication of application:
**16.02.83 Bulletin 83/07**

(45) Publication of the grant of the patent:
**25.11.87 Bulletin 87/48**

(84) Designated Contracting States:
**CH DE GB LI NL SE**

(56) References cited:
**DE-A-2 930 663**

(73) Proprietor: **SUMITOMO ELECTRIC INDUSTRIES LIMITED**
**No. 15, Kitahama 5-chome Higashi-ku**
**Osaka-shi Osaka 541 (JP)**

(72) Inventor: **Shiga, Tetsuya**
**c/o Osaka Works Of Sumitomo Electric, Ltd.**
**1-3, Shimaya 1-chome Konohana-ku Osaka (JP)**
Inventor: **Okawa, Shinichi**
**c/o Osaka Works Of Sumitomo Electric, Ltd.**
**1-3, Shimaya 1-chome Konohana-ku Osaka (JP)**
Inventor: **Yoshida, Kenichi**
**c/o Osaka Works Of Sumitomo Electric, Ltd.**
**1-3, Shimaya 1-chome Konohana-ku Osaka (JP)**
Inventor: **Hiramoto, Junichi**
**c/o Osaka Works Of Sumitomo Electric, Ltd.**
**1-3, Shimaya 1-chome Konohana-ku Osaka (JP)**

(74) Representative: **Henkel, Feiler, Hänzel & Partner**
**Möhlstrasse 37**
**D-8000 München 80 (DE)**

Courier Press, Leamington Spa, England.

## Description

The present invention relates to a device for the measurement of cardio-pulmonary functions. These functions comprise: $PO_2$, $PCO_2$, respiration rate and encardiography. Among the functions the first two are relatively new while the latter items have been practiced for a long time.

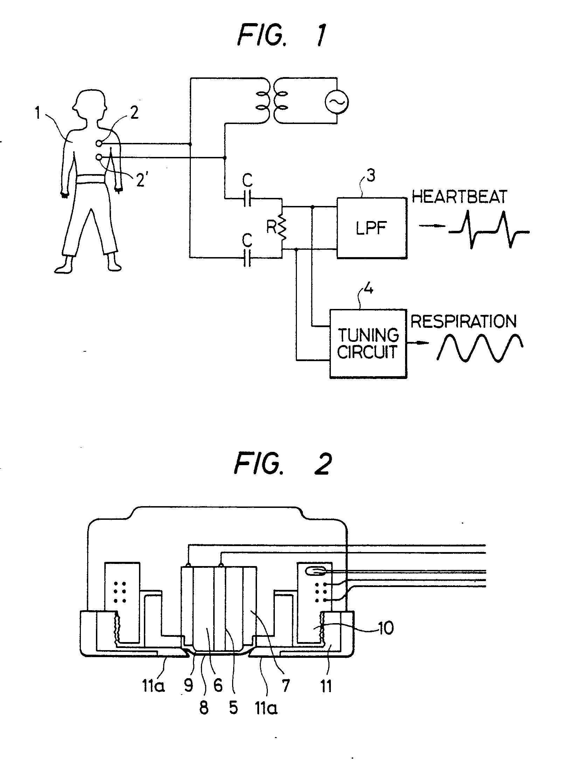

As is generally known, the respiration rate and encardiography are measured such that as shown in Figure 1, two electrodes 2, 2' are applied to predetermined portions on the skin of a human body 1 and fluctuations in the potential between the electrodes 2, 2' are taken as a measure of encardiography, while fluctuations in the impedance between these electrodes (2, 2') are used to measure the respiration rate.

Signals related to the two mentioned functions are led to a resistor R, and signals related to the heartbeat (of low frequency) are taken out by means of a low-pass filter 3 connected across the resistor R, while an alternate source of 50 KHz supplied for measurement is taken out by means of a tuning circuit 4 and detected in order to measure the respiration rate. Accordingly, two electrodes 2, 2' must be applied to the surface of the human body 1.

The transcutaneous measurement of $PO_2$ and $PCO_2$ requires the attachment of a $PO_2$ sensitive polarographic sensor and a pH sensitive $PCO_2$ sensor, respectively, on the surface of the body.

Prior art document DE—A—29 30 663 discloses a device for the measurement of cardio-pulmonary functions comprising an electrode assembly for the transcutaneous measurement of partial gas pressure in arterial blood. This electrode assembly includes an electrode part having an anode or reference electrode, a cathode or measuring electrode having a working surface, and an electrode holder of thermally and electrically insulating material for holding said anode electrode and said cathode electrode apart from one another in insulated relation. A membrane holder part has a blood gas permeable membrane thereon, and a skin heating part has an electrical heater, and a heat conducting metal block to which said heater is heat-conductively connected, said heat conducting metal block having a skin-contact surface. Finally, this known device has electrodes for measuring at least one of the respiration rate and heartbeat.

It is the object of the present invention to provide an improved sensor device for measurement of the four above mentioned cardio-pulmonary functions which enables simultaneous and continuous measurement of the human body in a non-invasive manner.

The present invention provides a device for the measurement of cardio-pulmonary functions, comprising: first means for the transcutaneous measurement of a blood gas partial pressure, said first means including a heat conducting member for transferring heat from an electrical heater to a patient's skin, and second means for measuring at least one of the heartbeat and respiration rate, said second means including an electrically conducting electrode, said device being characterized in that said electrode is comprised in said heat conducting member.

Furthermore, the present invention also provides a device for the simultaneous and continuous measurement of the cardio-pulmonary functions of the human body, comprising:

(a) an electrode assembly for the transcutaneous measurement of partial gas pressure in arterial blood comprising the following three removably assembled parts:

an electrode part having an anode or reference electrode having a ring-shaped working surface, a cathode or measuring electrode having a working surface inside said anode or reference electrode, and an electrode holder of thermally and electrically insulating material for holding said anode or reference electrode and said cathode or measuring electrode apart from one another in insulated relation,

a membrane holder part having a blood gas permeable membrane thereon and a membrane holder for holding the periphery of said membrane, and

a skin heating part having an electrical heater, and a heat conducting metal block to which said heater is heat-conductively connected, said heat-conducting metal block having a skin-contact surface,

said device being characterized by:

(b) means for measuring of at least one of the respiration rate and heartbeat connected to said skin-contact surface, said surface comprising an electrode of said measuring means.

As previously mentioned, each of the $PO_2$ and $PCO_2$ sensors uses a skin-heating metal plate in order to obtain a sufficient and stable state of vasodilatation of the tissues under the surface of the skin. According to the present invention such skin-heating plate is utilized as an electrode for measuring the respiration rate and encardiography, so that the four functions can be determined by means of only two electrodes or sensors. Thus, the present invention provides a sensing device for monitoring the cardio-pulmonary functions of a patient, especially new born babies in an intensive care unit. With the aid of this device up to four functions or parameters, i.e. $PO_2$, $PCO_2$, respiration rate and cardiography are measured in both a simultaneous and transcutaneous way.

This invention can be more fully understood from the following detailed description when taken in conjunction with the accompanying drawings, in which:

Figure 1 is a circuit diagram of a device for measuring heartbeat and respiration;

Figure 2 is a sectional diagram of a first preferred embodiment of the present invention;

Figure 3 is a circuit diagram of a second preferred embodiment of the present invention.

Figure 2 shows a sectional view of typical $PO_2$ non-invasive sensor assembly; A rod-shaped cathode 5 is disposed in the center, and an anode 7 is mounted about the cathode 5 through glass insulation 6. The ends of the electrodes contact an electrolyte 9 which is covered by an electrode membrane 8. The peripheral portion of the electrode 7 is covered by a metal heating ring 10 and a skin-heating metal plate 11. An exposed portion of the skin-heating metal plate 11 is made to contact the skin surface when the sensor assembly is attached to the surface of the body.

As described above, four electrodes need be attached to the surface of the human body when the above four functions are to be measured simultaneously. When such a large number of electrodes and sensors are required to be attached to a limited surface area of the patient, it often happens that some of the functions must be measured later due to space limitation. In addition, as the number of electrodes or sensors to be attached increases, the numbers of electric lead wires will naturally increase and cause difficulties in the handling of the electrodes or sensors especially in an intensive care unit.

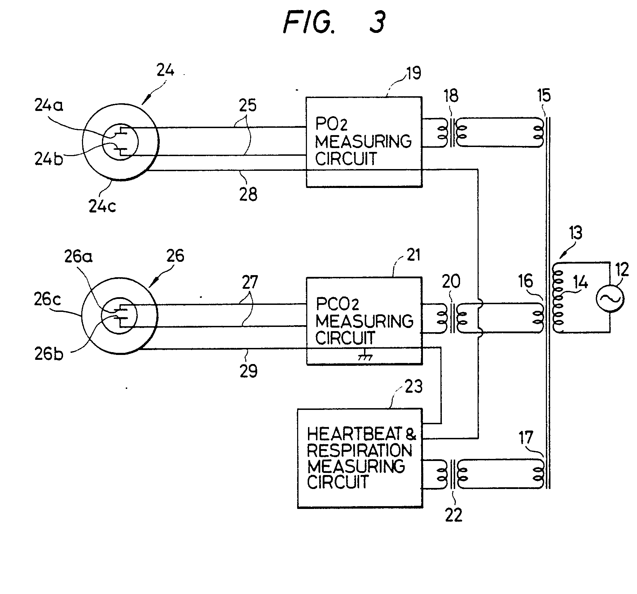

A preferred embodiment of the present invention will now be described with reference to Figure 3. In Figure 3, an AC power source 12 is connected to a primary coil 14 of a transformer 13. Three coils 15, 16 and 17 are wound on the secondary side of the transformer 13. The coil 15 is connected through a transformer 18 to a $PO_2$ measuring circuit 19, the coil 16 is connected through a transformer 20 to a $PCO_2$ measuring circuit 21, and the coil 17 is connected through a transformer 22 to a heartbeat and respiration measuring circuit 23. As the $PO_2$ measuring circuit 19, the $PCO_2$ measuring circuit 21 and the heartbeat and respiration measuring circuit 23, conventional circuits may be used, respectively. Reference numeral 24 designates the $PO_2$ sensor shown in Fig. 2. Electrodes 24a, 24b are connected to the $PO_2$ measuring circuit 19 through lead wires 25, 25 respectively. Similarly, electrodes 26, 26b of the $PCO_2$ sensor 26, which has the same construction as the above mentioned $PO_2$ sensor 24, are connected to the $PCO_2$ measuring circuit 21 through lead wires 27, 27 respectively. Rings 24c, 26c, illustrated by circles drawn about the electrodes of the $PO_2$ sensor 24 and the $PCO_2$ sensor 26 are similar to the skin-heating metal plates 11 shown in Fig. 2, and are connected to the heartbeat and respiration measuring circuit 23 through lead wires 28, 29 respectively. In other words, the rings 24c, 26c serving as skin-heating metal plates also function as the electrodes 2, 2 shown in Fig. 1. There are no particular differences in electrical operation between the rings 24c, 26c and conventional ones.

In conducting measurement using the preferred embodiment, the $PO_2$ sensor 24 and the $PCO_2$ sensor 26 are attached at positions suitable for measuring heartbeat and respiration. $PO_2$ is then measured through the electrodes 24a, 24b while $PCO_2$ is measured through the electrodes 26a, 26b as usual. At the same time, heartbeat and respiration can be measured using the rings 24c, 26c as electrodes. In other words, it becomes possible to conduct the measurement of four items simultaneously by attaching only two sensors.

For the lead wires 25, 25 and 28, a three-core cord can be used, and for the lead wires 27, 27 and 29 a second three-core cord can be used likewise. The number of cords thus decreases, so that handling is facilitated.

As mentioned above, according to the present invention, it is possible to conduct the measurement of four functions simultaneously using only two electrodes. In addition, the handling of the equipment is facilitated due to the decreased number of electrodes or sensor lead wires.

## Claims

1. A device for the measurement of cardio-pulmonary functions, comprising:

first means (19, 21) for the transcutaneous measurement of a blood gas partial pressure, said first means (19, 21) including a heat conducting member (24c, 26c) for transferring heat from an electrical heater to a patient's skin, and

second means (23) for measuring at least one of the heartbeat and respiration rate, said second means (23) including an electrically conducting electrode (2),

characterized in that said electrode (2) is comprised in said heat conducting member (24c, 26c).

2. A device as claimed in claim 1, characterized in that said first means (19, 21) comprise one of a $PO_2$ and $PCO_2$ sensor.

3. A device as claimed in claim 1 or 2, characterized in that said second means (23) include circuit means for measuring the heartbeat and respiration rate, and are electrically connected to said heat conducting member (24c, 26c).

4. A device as claimed in any one of claims 1 to 3, characterized in that said first means (19, 21) comprise one of a $PO_2$ and $PCO_2$ sensor including an electrode pair (24a, 24b; 26a, 26b) electrically connected to a measuring circuit by a pair of lead wires (25, 27), and said second means (23) include a respiration and heartbeat measuring circuit electrically connected to said heat conducting member (24c, 26c) by a further lead wire (28, 29).

5. A device as claimed in claim 4, characterized in that said pair of lead wires (25, 27, 28, 29) and said further lead wire together constitute a three-core cord extending from said sensor to said respective measuring circuits.

6. A device for the simultaneous and continuous measurement of the cardio-pulmonary functions of the human body, comprising:

(a) an electrode assembly for the transcutaneous measurement of partial gas pressure in arterial blood comprising the following three removably assembled parts:

an electrode part having an anode (7) or reference electrode having a ring-shaped working surface, a cathode (5) or measuring electrode having a working surface inside said anode (7) or reference electrode, and an electrode holder (6) of thermally and electrically insulating material for holding said anode (7) or reference electrode and said cathode (5) or measuring electrode apart from one another in insulated relation,

a membrane holder part having a blood gas permeable membrane (8) thereon and a membrane holder for holding the periphery of said membrane, and

a skin heating part (10) having an electrical heater, and a heat conducting metal block (11) to which said heater is heat-conductively connected, said heat conducting metal block (11) having a skin-contact surface (11a),

characterized by

(b) means for measuring of at least one of the respiration rate and heartbeat connected to said skin-contact surface (11a), said surface comprising an electrode of said measuring means.

## Patentansprüche

1. Vorrichtung zur Messung kardio-pulmonaler Funktionen, mit:

einer ersten Einrichtung (19, 21) zur transkutanen Messung eines Blutgaspartialdruckes, wobei die erste Einrichtung (19, 21) ein Wärmeleiterglied (24c, 26c) zum Übertragen von Wärme von einer elektrischen Heizeinheit zu einer Patientenhaut umfaßt, und

einer zweiten Einrichtung (23) zum Messen wenigstens des Herzschlagens und/oder der Atmungsrate, wobei die zweite Einrichtung (23) eine elektrisch leitende Elektrode (2) umfaßt, dadurch gekennzeichnet, daß die Elektrode (2) in dem Wärmeleiterglied (24c, 26c) vorgesehen ist.

2. Vorrichtung nach Anspruch 1, dadurch gekennzeichnet, daß die erste Einrichtung (19, 21) einen PO₂-Sensor oder einen PCO₂-Sensor umfaßt.

3. Vorrichtung nach Anspruch 1 oder 2, dadurch gekennzeichnet, daß die zweite Einrichtung (23) eine Schaltungseinrichtung zum Messen des Herzschlagens und der Atmungsrate umfaßt und elektrisch mit dem Wärmeleiterglied (24c, 26c) verbunden ist.

4. Vorrichtung nach einem der Ansprüche 1 bis 3, dadurch gekennzeichnet, daß die erste Einrichtung (19, 21) einen PO₂-Sensor oder einen PCO₂-Sensor einschließlich eines elektrisch mit einer Meßschaltung durch zwei Leitungsdrähte (25, 27) verbundenen Elektrodenpaares (24a, 24b; 26a, 26b) umfaßt, und daß die zweite Einrichtung (23) eine Atmung- und Herzschlagen-Meßschaltung aufweist, die elektrisch mit dem Wärmeleiterglied (24c, 26c) durch einen weiteren Leitungsdraht (28, 29) verbunden ist.

5. Vorrichtung nach Anspruch 4, dadurch gekennzeichnet, daß die beiden Leitungsdrähte (25, 27, 28, 29) und der weitere Leitungsdraht zusammen ein dreiadriges Kabel bilden, das sich vom Sensor zu den jeweiligen Meßschaltungen erstreckt.

6. Vorrichtung für die gleichzeitige und kontinuierliche Messung der kardio-pulmonalen Funktionen des menschlichen Körpers, mit

(a) einer Elektrodenanordnung für die transkutane Messung des Partialgasdruckes im arteriellen Blut aus den folgenden drei entfernbar zusammengebauten Teilen:

einem Elektrodenteil mit einer Anode (7) oder Bezugselektrode mit einer ringförmigen Arbeitsfläche, einer Kathode (5) oder Meßelektrode mit einer Arbeitsfläche innerhalb der Anode (7) oder Bezugselektrode und einem Elektrodenhalter (6) aus einem thermisch und elektrisch isolierendem Material, um die Anode (7) oder die Bezugselektrode und die Kathode (5) oder Meßelektrode von einander in isolierter Beziehung beabstandet zu halten,

einem Membranhalterteil mit einer blutgaspermeablen Membran (8) darauf und einem Membranhalter zum Halten des Randes der Membran, und

einem Hauterwärmungsteil (10) mit einer elektrischen Heizeinheit und einem Wärmeleitermetallblock (11), mit dem die Heizeinheit wärmeleitend verbunden ist, wobei der Wärmeleitermetallblock (11) eine Hautkontaktfläche (11a) hat, gekennzeichnet durch

(b) eine Einrichtung zum Messen wenigstens der Atmungsrate und/oder des Herzschlagens, die mit der Hautkontaktfläche (11a) verbunden ist, welche eine Elektrode der Meßeinrichtung aufweist.

## Revendications

1. Dispositif pour mesurer des fonctions cardiopulmonaires, comportant:

des premiers moyens (19, 21), pour la mesure transcutanée d'une pression partielle de gaz dans le sang, ces premiers moyens (19, 21) comprenant un élément conducteur de la chaleur (24c, 26c) pour transmettre la chaleur d'un organe de chauffage électrique à la peau d'un patient, et

des seconds moyens (23) pour mesurer au moins le rythme cardiaque ou le rythme respiratoire, ces seconds moyens (23) comprenant une électrode (2) électriquement conductrice, caractérisé en ce que l'électrode précitée (2) est incorporée dans l'élément conducteur de la chaleur (24c, 26c).

2. Dispositif selon la revendication 1, caractérisé en ce que les premiers moyens (19, 21) comportent un capteur pour mesurer PO₂ ou PCO₂.

3. Dispositif selon la revendication 1 ou 2, caractérisé en ce que les seconds moyens (23) comportent un circuit pour mesurer le rythme cardiaque et le rythme de respiration, et sont reliés électriquement à l'élément conducteur de la chaleur (24c, 26c).

4. Dispositif selon l'une des revendications 1 à 3, caractérisé en ce que les premiers moyens (19, 21) comportent un capteur pour mesurer PO₂ et/ou PCO₂, et une paire d'électrodes (24a, 24b; 26a, 26b) électriquement reliées à un circuit de mesure par une paire de fils conducteurs (25, 27); et que les seconds moyens (23) incluent un circuit pour mesurer le rythme respiratoire et le rythme cardiaque, électriquement relié à l'élément conducteur de la chaleur (24c, 26c) par un autre fil conducteur (28, 29).

5. Dispositif selon la revendication 4, caractérisé en ce que la paire précitée de fils conducteurs (25, 27, 28, 29) et l'autre fil conducteur constituent ensemble un cordon à trois conducteurs allant du capteur précité auxdits circuits de mesure respectifs.

6. Dispositif pour réaliser la mesure simultanée et continue des fonctions cardio-pulmonaires du corps humain, comportant:

(a) un ensemble à électrodes pour la mesure transcutanée d'une pression gazeuse partielle dans le sang artériel, constitué par les trois parties ci-après, réunies de manière amovible:

une électrode pourvue d'une anode (7), ou électrode de référence, présentant une surface active de forme annulaire, une cathode (5), ou électrode de mesure, présentant une surface active disposée à l'intérieur de l'anode (7), ou électrode de référence, et une monture (6) en un matériau thermiquement et électriquement isolant, pour maintenir écartées et isolées l'une de l'autre l'anode (7), ou électrode de référence, et la cathode (5), ou électrode de mesure,

un support de membrane, pourvu d'une membrane (8), perméable aux gaz contenus dans le sang et fixée par sa périphérie audit support, et

un organe de chauffage de la peau (10), pourvu d'un élément de chauffage électrique et d'un bloc métallique (11) conducteur de la chaleur auquel l'organe de chauffage est thermiquement relié, ce bloc métallique conducteur de la chaleur (11) présentant une face (11a) adaptée à venir au contact de la peau,

dispositif caractérisé en ce qu'il comporte:

(b) des moyens pour mesurer le rythme respiratoire et/ou le rythme cardiaque, ces moyens étant reliés à la face (11a) du bloc métallique adaptée à venir au contact de la peau, et dans laquelle est incluse une électrode qui partie desdits moyens de mesure.

## FIG. 1

## FIG. 2

0 071 980

# FIG. 3

2